# EUROPEAN PATENT APPLICATION

(11) **EP 4 631 495 A1**
(43) Date of publication of application: **15.10.2025**
(21) Application number: 23899991.6
(22) Date of filing: 06.12.2023
(51) Int. Cl.: A61K 8/9789, A61K 8/9783, A61K 8/97, A61K 8/92, A61Q 19/02, A61Q 19/00

(54) **LIGHTENING AND MOISTURIZING SKIN CARE PRODUCT COMPOSITION COMPRISING RADIX ASTRAGALI AND USE THEREOF**

(30) Priority: 07.12.2022 CN 202211564197
(71) Applicant: Sichuan Baili Pharmaceutical Co. Ltd., Wenjiang District Chengdu Sichuan 611130 (CN)
(72) Inventor: ZHU, Yi, Chengdu, Sichuan 611130 (CN); CHEN, Yi, Chengdu, Sichuan 611130 (CN); ZHANG, Wei, Chengdu, Sichuan 611130 (CN); LIU, Xiaoli, Chengdu, Sichuan 611130 (CN); XIAO, Xue, Chengdu, Sichuan 611130 (CN)
(74) Representative: Ghirardi, Valeria
(86) International application number: PCT/CN2023/136632
(87) International publication number: WO 2024/120417

(57) **Abstract**

The present invention relates to a lightening and moisturizing skin care product composition comprising *Radix Astragali* and use thereof. The skin care product composition comprises 1-15 parts by weight of a *Radix Astragali* extract, 1-5 parts by weight of an aloe extract, 1-5 parts by weight of a green tea extract, 0.1-5 parts by weight of a thickener, and 0.1-3 parts by weight of a preservative. The skin care product composition has the effects of improving the skin state and lightening and moisturizing the skin.

## Description

### Technical Field

The present invention belongs to the field of skin care products, and specifically relates to a lightening and moisturizing skin care product composition comprising *Radix Astragali* and use thereof.

### Background Art

The skin is the largest organ of the human body exposed to the outside world, and plays a role in protecting the human body from external stimulation or damage. The skin has a barrier function, preventing the loss of water, electrolytes and other substances from the body, and preventing the invasion of harmful substances from the outside world.

With the increase of age, the skin is affected by ultraviolet rays and pollutants in the air, and is prone to lack of nutrition, which in turn causes the skin to lose water, destroys the keratin of the skin, and causes the skin to be rough, dull and pigmented. Therefore, it is necessary to provide a skin care product that can enhance skin nutrition, maintain skin moisture, and lighten and brighten the skin.

### Contents of the present invention

The present invention provides a lightening and moisturizing skin care product composition comprising *Radix Astragali,* which has the effects of improving skin condition, lightening and moisturizing skin.

To this end, in the first aspect of the present invention, the present invention provides a skin care product composition, which comprises:
1 to 15 parts by weight (e.g., 1, 2, 3, 4, 5, 6, 7, 8, 9, 10, 11, 12, 13, 14 or 15 parts by weight) of *Radix Astragali* extract;
1 to 5 parts by weight (e.g., 1, 1.5, 2, 2.5, 3, 3.5, 4, 4.5 or 5 parts by weight) of aloe extract;
1 to 5 parts by weight (e.g., 1, 1.5, 2, 2.5, 3, 3.5, 4, 4.5 or 5 parts by weight) of green tea extract;
0.1 to 5 parts by weight (e.g., 0.1, 0.5, 1, 1.5, 2, 2.5, 3, 3.5, 4, 4.5 or 5 parts by weight) of thickening agent;
0.1 to 3 parts by weight (e.g., 0.1, 0.5, 1, 1.5, 2, 2.5 or 3 parts by weight) of preservative.

In some embodiments, the skin care product composition further comprises:
0.1 to 40 parts by weight (e.g., 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 11.7, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 31.5, 32, 32.5, 33, 33.5, 34, 34.5, 35, 35.5, 36, 36.5, 37, 37.5, 38, 38.5, 39, 39.5 or 40 parts by weight) of moisturizing agent;
0.1 to 30 parts by weight (e.g., 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 or 30 parts by weight) of lightening agent;
0.1 to 10 parts by weight (e.g., 0.1, 0.5, 1, 1.5, 2, 2.5, 3, 3.5, 4, 4.5, 5, 5.5, 6, 6.5, 7, 7.5, 8, 8.5, 9, 9.5 or 10 parts by weight) of permeation enhancer.

In some embodiments, the skin care product composition comprises:
4 to 12 parts by weight (e.g., 4, 4.5, 5, 5.5, 6, 6.5, 7, 7.5, 8, 8.5, 9, 9.5, 10, 10.5, 11, 11.5 or 12 parts by weight) of *Radix Astragali* extract;
1 to 2 parts by weight (e.g., 1, 1.5 or 2 parts by weight) of aloe extract;
1 to 2 parts by weight (e.g., 1, 1.5 or 2 parts by weight) of green tea extract;
0.1 to 5 parts by weight (e.g., 0.1, 0.5, 1, 1.5, 2, 2.5, 3, 3.5, 4, 4.5 or 5 parts by weight) of thickening agent;
0.1 to 3 parts by weight (e.g., 0.1, 0.5, 1, 1.5, 2, 2.5 or 3 parts by weight) of preservative;
5 to 30 parts by weight (e.g., 5, 5.5, 6, 6.5, 7, 7.5, 8, 8.5, 9, 9.5, 10, 10.5, 11, 11.5, 11.7, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 or 30 parts by weight) of moisturizing agent;
1 to 10 parts by weight (e.g., 1, 1.5, 2, 2.5, 3, 3.5, 4, 4.5, 5, 5.5, 6, 6.5, 7, 7.5, 8, 8.5, 9, 9.5 or 10 parts by weight) of lightening agent;
1 to 7 parts by weight (e.g., 1, 1.5, 2, 2.5, 3, 3.5, 4, 4.5, 5, 5.5, 6, 6.5 or 7 parts by weight) of permeation enhancer.

In some embodiments, the skin care product composition per 100 parts by weight consists of:
4 to 12 parts by weight (e.g., 4, 4.5, 5, 5.5, 6, 6.5, 7, 7.5, 8, 8.5, 9, 9.5, 10, 10.5, 11, 11.5 or 12 parts by weight) of *Radix Astragali* extract;
1 to 2 parts by weight (e.g., 1, 1.5 or 2 parts by weight) of aloe extract;
1 to 2 parts by weight (e.g., 1, 1.5 or 2 parts by weight) of green tea extract;
0.1 to 5 parts by weight (e.g., 0.1, 0.5, 1, 1.5, 2, 2.5, 3, 3.5, 4, 4.5 or 5 parts by weight) of thickening agent;
0.1 to 3 parts by weight (e.g., 0.1, 0.5, 1, 1.5, 2, 2.5 or 3 parts by weight) of preservative;
5 to 30 parts by weight (e.g., 5, 5.5, 6, 6.5, 7, 7.5, 8, 8.5, 9, 9.5, 10, 10.5, 11, 11.5, 11.7, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 or 30 parts by weight) of moisturizing agent;
1 to 10 parts by weight (e.g., 1, 1.5, 2, 2.5, 3, 3.5, 4, 4.5, 5, 5.5, 6, 6.5, 7, 7.5, 8, 8.5, 9, 9.5 or 10 parts by weight) of lightening agent;
1 to 7 parts by weight (e.g., 1, 1.5, 2, 2.5, 3, 3.5, 4, 4.5, 5, 5.5, 6, 6.5 or 7 parts by weight) of permeation enhancer; and
water as the remainder.

In some embodiments, the skin care product composition further comprises:
0.1 to 10 parts by weight (e.g., 0.1, 0.5, 1, 1.5, 2, 2.5, 3, 3.5, 4, 4.5, 5, 5.5, 6, 6.5, 7, 7.5, 8, 8.5, 9, 9.5 or 10 parts by weight; preferably 1 to 7 parts by weight) of emulsifying agent.

In some embodiments, the moisturizing agent comprises:
0.1 to 30 parts by weight (e.g., 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 11.7, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 or 30 parts by weight; preferably 5 to 20 parts by weight) of polyol moisturizing agent,
0.1 to 15 parts by weight (e.g., 0.1, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 11.7, 12, 12.5, 13, 13.5, 14, 14.5 or 15 parts by weight; preferably 0.1 to 10 parts by weight) of carbohydrate moisturizing agent, and
0.1 to 30 parts by weight (e.g., 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 11.7, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 or 30 parts by weight; preferably 0.2 to 2.5 parts by weight; more preferably 0.2 to 1 parts by weight) of other moisturizing agent.

In some embodiments, the polyol moisturizing agent is selected from the group consisting of glycerin, 1,3-butanediol, sorbitol, polyethylene glycol, propylene glycol, glycereth-26, and any combination thereof.

In some embodiments, the polyol moisturizing agent is selected from the group consisting of glycerin, 1,3-butanediol, glycereth-26, and any combination thereof.

In some embodiments, the polyol moisturizing agent is a composition consisting of 5 to 12 parts by weight (e.g., 5, 5.5, 6, 6.5, 7, 7.5, 8, 8.5, 9, 9.5, 10, 10.5, 11, 11.5, 11.7 or 12 parts by weight) of glycerin and 2 to 8 parts by weight (e.g., 2, 2.5, 3, 3.5, 4, 4.5, 5, 5.5, 6, 6.5, 7, 7.5 or 8 parts by weight) of 1,3-butanediol, or a composition consisting of 5 to 12 parts by weight (e.g., 5, 5.5, 6, 6.5, 7, 7.5, 8, 8.5, 9, 9.5, 10, 10.5, 11, 11.5, 11.7 or 12 parts by weight) of glycerin, 2 to 8 parts by weight (e.g., 2, 2.5, 3, 3.5, 4, 4.5, 5, 5.5, 6, 6.5, 7, 7.5 or 8 parts by weight) of 1,3-butanediol, and 1.5 to 2.5 parts by weight (preferably 2 parts by weight) of glycereth-26.

In some embodiments, the carbohydrate moisturizing agent is selected from the group consisting of sodium hyaluronate, trehalose, tremella polysaccharide, methyl glucose polyether, and any combination thereof.

In some embodiments, the carbohydrate moisturizing agent is selected from the group consisting of sodium hyaluronate, trehalose, and any combination thereof.

In some embodiments, the carbohydrate moisturizing agent is a composition consisting of 0.5 to 8 parts by weight (e.g., 0.5, 0.6, 0.7, 0.8, 0.9, 1, 1.5, 2, 2.5, 3, 3.5, 4, 4.5, 5, 5.5, 6, 6.5, 7, 7.5 or 8 parts by weight) of sodium hyaluronate, or a composition consisting of 0.5 to 8 parts by weight (e.g., 0.5, 0.6, 0.7, 0.8, 0.9, 1, 1.5, 2, 2.5, 3, 3.5, 4, 4.5, 5, 5.5, 6, 6.5, 7, 7.5 or 8 parts by weight) of sodium hyaluronate and 1.5 to 2.5 parts by weight (preferably 2 parts by weight) of trehalose.

In some embodiments, the other moisturizing agent is selected from the group consisting of panthenol, ceramide, allantoin, natural plant extract, and any combination thereof.

In some embodiments, the other moisturizing agent is allantoin.

In some embodiments, the other moisturizing agent consists of 0.2 to 1 parts by weight (e.g., 0.2, 0.3, 0.4, 0.5, 0.6, 0.7, 0.8, 0.9 or 1 parts by weight) of allantoin.

In some embodiments, the moisturizing agent further comprises:
0.1 to 8 parts by weight (e.g., 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5 or 8 parts by weight; preferably 0.1 to 6 parts by weight) of natural moisturizing factor moisturizing agent.

In some embodiments, the natural moisturizing factor moisturizing agent is selected from the group consisting of urea, amino acid, vitamin B6, and any combination thereof.

In some embodiments, the thickening agent is selected from the group consisting of xanthan gum, carbomer 940, polyacrylic acid polymer, and any combination thereof.

In some embodiments, the thickening agent is xanthan gum.

In some embodiments, the preservative is selected from the group consisting of paraben (e.g., methyl paraben, ethyl paraben, propyl paraben, butyl paraben), phenoxyethanol, benzoic acid/benzyl alcohol and derivatives thereof, and any combination thereof.

In some embodiments, the preservative is phenoxyethanol.

In some embodiments, the lightening agent is selected from the group consisting of hydroquinone, arbutin, vitamin C and derivatives thereof (e.g., vitamin C, ascorbyl glucoside), niacinamide, natural plant extract, and any combination thereof.

In some embodiments, the lightening agent is selected from the group consisting of arbutin, vitamin C and derivatives thereof (e.g., vitamin C, ascorbyl glucoside), niacinamide, natural plant extract, and any combination thereof.

In some embodiments, the lightening agent is selected from the group consisting of arbutin, vitamin C and derivatives thereof (e.g., vitamin C, ascorbyl glucoside), niacinamide, and any combination thereof.

In some embodiments, the arbutin is α-arbutin.

In some embodiments, the permeation enhancer is selected from the group consisting of Azone, isosorbide dimethyl ether, inositol, isopropyl myristate, and any combination thereof.

In some embodiments, the permeation enhancer is isopropyl myristate.

In some embodiments, the emulsifying agent is selected from the group consisting of glyceryl stearate, methyl glucose sesquistearate, PEG-20 methyl glucose sesquistearate, laureth-7/C₁₃₋₁₄ alkane/polyacrylamide (also known as Emulsifier 305), isopropyl myristate, and any combination thereof.

In some embodiments, the skin care product composition further comprises an essence.

In some embodiments, the essence is selected from the group consisting of bluebell essence, Miss COCO essence, cherry blossom essence, ebony agarwood essence, jasmine essence, rose essence, and grapefruit essential oil.

In some embodiments, the essence is present in an amount of 0.01 to 0.02 parts by weight (e.g., 0.011, 0.012, 0.013, 0.014, 0.015, 0.016, 0.017, 0.018, 0.019 or 0.02 parts by weight).

In some embodiments, the *Radix Astragali* extract is obtained by the following method, comprising: 1) taking *Radix Astragali,* adding water and performing decoction extraction, discarding the residue, and obtaining a medicinal liquid; 2) concentrating the medicinal liquid to obtain a clear paste; 3) mixing the clear paste with ethanol for alcohol precipitation to obtain a precipitate, and taking the precipitate as the *Radix Astragali* extract.

In some embodiments, the *Radix Astragali* extract is obtained by the following method, comprising: 1-1) taking *Radix Astragali,* adding water and performing the first decoction extraction to obtain a medicinal residue and a medicinal liquid; 1-2) adding water to the medicinal residue, performing the second decoction extraction, discarding the medicinal residues, and obtaining a medicinal liquid; 2) combining the two medicinal liquids obtained by the two decoction extraction steps, and performing concentration to obtain a clear paste; 3) mixing the clear paste with ethanol for alcohol precipitation to obtain a precipitate, and taking the precipitate as the *Radix Astragali* extract.

In some embodiments, in the first decoction extraction, the weight of the added water is 1 to 10 times (e.g., 1, 2, 3, 4, 5, 6, 7, 8, 9 or 10 times) the weight of the *Radix Astragali.*

In some embodiments, in the second decoction extraction, the weight of the added water is 1 to 10 times (e.g., 1, 2, 3, 4, 5, 6, 7, 8, 9 or 10 times) the weight of the medicinal residue.

In some embodiments, the first decoction extraction is performed for 1 to 10 hours (e.g., 1, 2, 3, 4, 5, 6, 7, 8, 9 or 10 hours).

In some embodiments, the second decoction extraction is performed for 1 to 10 hours (e.g., 1, 2, 3, 4, 5, 6, 7, 8, 9 or 10 hours).

In some embodiments, the clear paste has a relative density of 1.21 to 1.24 (60°C thermal measurement).

In some embodiments, the mixed solution after the clear paste is mixed with ethanol has an ethanol content of 70%.

In some embodiments, the method further comprises: 4) drying, pulverizing, and sterilizing the precipitate.

Those skilled in the art can understand that "decoction" refers to boiling *Radix Astragali* after adding water.

In some embodiments, the aloe extract is obtained by the following steps: 1) taking aloe, washing, air-drying, peeling, de-enzyming, baking, crushing and sieving to obtain an aloe powder, adding anhydrous ethanol to the aloe powder for soaking, performing extraction under microwave radiation, filtering to obtain a first extract solution, recovering solvent to obtain a first concentrated solution, and taking the filter residue for later use; 2) adding water-saturated n-butanol solution to the filter residue for soaking, performing ultrasonic extraction, filtering to obtain a second extract solution, recovering solvent to obtain a second concentrated solution; 3) mixing the first concentrated solution and the second concentrated solution, and drying to obtain the aloe extract.

In some embodiments, the green tea extract is obtained by the following steps: taking green tea, using water as solvent, heating to 80°C in a water bath and extracting for multiple times; combining the extract solutions, then performing extraction with an equal volume of chloroform, separating the chloroform phase, extracting the retained aqueous phase with ethyl acetate for multiple times, recovering most of the ethyl acetate and concentrating the extract solution to near dryness, freeze-drying the concentrate and repeatedly recrystallizing with deionized water to obtain the green tea extract.

In some embodiments, the skin care product composition consists of:

| Ingredient | Amount (%) |
|---|---|
| *Radix Astragali* extract | 5 |
| aloe extract | 2 |
| green tea extract | 2 |
| sodium hyaluronate | 0.5 |
| glycerin | 5 |
| 1,3-butanediol | 2 |
| trehalose | 2 |
| glycereth-26 | 2 |
| allantoin | 0.2 |
| niacinamide | 1 |
| α-arbutin | 0.5 |
| ascorbyl glucoside | 0.5 |
| xanthan gum | 1.5 |
| phenoxyethanol | 0.5 |
| isopropyl myristate | 3 |
| grapefruit essential oil | 0.015 |
| water | balance |

or,

| Ingredient | Amount (%) |
|---|---|
| *Radix Astragali* extract | 12 |
| aloe extract | 2 |
| green tea extract | 2 |
| sodium hyaluronate | 0.5 |
| glycerin | 10 |
| 1,3-butanediol | 8 |
| allantoin | 1 |
| niacinamide | 1 |
| α-arbutin | 0.5 |
| ascorbyl glucoside | 0.5 |
| xanthan gum | 1 |
| phenoxyethanol | 0.5 |
| isopropyl myristate | 5 |
| grapefruit essential oil | 0.015 |
| water | balance |

or,

| Ingredient | Amount (%) |
|---|---|
| *Radix Astragali* extract | 4 |
| aloe extract | 1 |
| green tea extract | 1 |
| sodium hyaluronate | 8 |
| glycerin | 5 |
| 1,3-butanediol | 5 |
| allantoin | 0.5 |
| niacinamide | 5 |
| α-arbutin | 2 |
| ascorbyl glucoside | 2 |
| xanthan gum | 4 |
| phenoxyethanol | 2 |
| isopropyl myristate | 7 |
| grapefruit essential oil | 0.015 |
| water | balance |

or,

| Ingredient | Amount (%) |
|---|---|
| *Radix Astragali* extract | 10 |
| aloe extract | 2 |
| green tea extract | 2 |
| sodium hyaluronate | 0.5 |
| glycerin | 5 |
| 1,3-butanediol | 2 |
| trehalose | 2 |
| glycereth-26 | 2 |
| allantoin | 0.2 |
| niacinamide | 1 |
| α-arbutin | 0.5 |
| ascorbyl glucoside | 0.5 |
| xanthan gum | 1.5 |
| phenoxyethanol | 0.5 |
| isopropyl myristate | 3 |
| grapefruit essential oil | 0.015 |
| water | balance |

In the second aspect of the present invention, the present invention provides a skin care product, which comprises: the skin care product composition as described above.

In some embodiments, the skin care product is a facial mask.

In the third aspect of the present invention, the present invention provides a method for enhancing the effects of lightening and moisturizing the skin, which comprises:
applying the skin care product composition as described above or the skin care product as described above to the skin.

### Beneficial effects

1. The skin care product composition of the present invention has the effects of improving skin condition, lightening and moisturizing skin.
2. The skin care product composition of the present invention has the significant effects of lightening and rejuvenating skin, can increase skin smoothness and elasticity, reduce skin fine lines and wrinkles, keep skin moisturized, and increase skin firmness and elasticity.

### Specific Models for Carrying Out the present invention

The embodiments of the present invention will be described in detail below in conjunction with the examples, but those skilled in the art will understand that the following examples are only used to illustrate the present invention, rather than to limit the scope of the present invention. According to the following detailed description of the preferred embodiment, the various objects and advantages of the present invention will become apparent to those skilled in the art.

In the present invention, the amount (%) of each ingredient refers to the percentage of weight of each ingredient in the total weight of the skin care product composition.

Most of the reagents of the present invention were commercially available, and some were self-made. Exemplary sources were as follows:

| Ingredient | Source |
|---|---|
| *Radix Astragali* extract | Self-made, the preparation method was described below. |
| Aloe extract | Xi'an Lvtian Biotechnology Co., Ltd., 1PMA211101 |
| Green tea extract | Xi'an Dongchi Biotechnology Co., Ltd., DC-2021061002 |
| *Angelica dahurica* extract | Shaanxi Xintianyu Biotechnology Co., Ltd., XTY20220129 |
| *Ligusticum chuanxiong* extract | Shaanxi Xintianyu Biotechnology Co., Ltd., XTY20220113 |
| *Ganoderma lucidum* extract | Shaanxi Xintianyu Biotechnology Co., Ltd., XTY20220325 |
| Sodium hyaluronate | Bloomage Biotech, 21120142 |
| Glycerin | Chengdu Opal Trading Co., Ltd., 3A0561F |
| 1,3-Butanediol | Guangzhou Chenghao Trading Co., Ltd., 2020-12 |
| Trehalose | Chengdu Yilin Technology Co., Ltd., 1H021 |
| Glycereth-26 | Zhenyue (Guangdong) Technology Research and Development Co., Ltd., Z89226804 |
| Allantoin | Shaanxi Haochen Biotechnology Co., Ltd., 20211115 |
| Niacinamide | Shaanxi Haochen Biotechnology Co., Ltd., 0000197486 |
| α-Arbutin | Beijing Belliles Biotechnology Co., Ltd., 210501 |
| Ascorbyl glucoside | Xi'an Lvtian Biotechnology Co., Ltd., PMA210918 |
| Xanthan gum | Chengdu Yilin Technology Co., Ltd., 1-9-A |
| Phenoxyethanol | Zhuanglai Holdings (Suzhou) Co., Ltd., 20210325 |
| Isopropyl myristate | Zhejiang Wumei Biotechnology Co., Ltd., 2201002 |
| Grapefruit essential oil | Shanghai Zixinyuan Perfumery Co., Ltd., 2202513525 |

It should be noted that aloe extract, green tea extract, *Angelica dahurica* extract, *Ligusticum chuanxiong* extract, and *Ganoderma lucidum* extract could be purchased commercially or prepared by the following conventional methods.
1. Preparation process of *Radix Astragali* extract: *Radix Astragali* was taken and extracted twice (for the first time, 5 times the amount of water was added, and decoction was performed for 3 hours; for the second time, 3 times the amount of water was added, and decoction was performed for 2 hours), the extract solution was concentrated to obtain a clear paste (which had a relative density of 1.21~1.24 (60°C thermal measurement)), the clear paste was added with ethanol to perform alcohol precipitation (after ethanol was added to the clear paste, the ethanol content of the mixed solution was 70%) and allowed to stand, and the precipitate was dried, crushed and sterilized to obtain *Radix Astragali* extract.
2. Preparation process of aloe extract: 1) Aloe was taken, cleaned by washing, air-dried, peeled, de-enzymed, baked, crushed and sieved to obtain an aloe powder, the aloe powder was added with anhydrous ethanol and soaked, subjected to extraction under microwave radiation, and filtered to obtain a first extract solution, the solvent was recovered to obtain a first concentrated solution, and the filter residue was taken for later use; 2) the filter residue was added with water-saturated n-butanol solution and soaked, subjected to ultrasonic extraction, and filtered to obtain a second extract solution, the solvent was recovered to obtain a second concentrated solution; 3) the first concentrated solution and the second concentrated solution were mixed, and dried to obtain aloe extract.
3. Preparation process of green tea extract: Water was used as solvent, and extraction was performed for multiple times by using a water bath at 80°C. The resultant extract solutions were combined, and extracted with an equal volume of chloroform. After the chloroform phase was separated, the residual phase was extracted with ethyl acetate for multiple times. After most of the ethyl acetate was recovered, the extract solution was concentrated to near dryness. The concentrate was then freeze-dried and recrystallized repeatedly with deionized water to obtain green tea extract.
4. Preparation process of *Angelica dahurica* extract: *Angelica dahurica* was taken and added with water for distillation, and the aqueous solution obtained by distillation was collected. The residue after distillation was added with the aqueous solution obtained by distillation and water to perform decoction twice, the first time for 1.5 hours and the second time for 1 hour. The decoction solutions were filtered, and the filtrates were combined, concentrated and dried to obtain *Angelica dahurica* extract.
5. Preparation process of *Ligusticum chuanxiong* extract: *Ligusticum chuanxiong* was taken, added with 6 times the amount of water and decocted for 3 times, each time for 30 minutes. The decoction solutions of the 3 times were combined and filtered, concentrated under reduced pressure and dried to obtain *Ligusticum chuanxiong* extract.
6. Preparation process of *Ganoderma lucidum* extract: *Ganoderma lucidum* was cleaned by washing, sliced and crushed, then extracted for 3 times in total, in which the first two extractions were performed using ammonia solution (i.e., ammonia water with an ammonia content of 10% to 35%) for extraction, and the last extraction was performed using water for extraction. After filtering, the filtrates of the 3 times were combined, concentrated and dried to obtain *Ganoderma lucidum* extract.

The present invention will be further explained in conjunction with specific examples below.

### Example 1: Preparation of skin care product composition

| Ingredient | Amount (%) | Function |
|---|---|---|
| *Radix Astragali* extract | 5 | / |
| Aloe extract | 2 | / |
| Green tea extract | 2 | / |
| Sodium hyaluronate | 0.5 | Moisturizing agent |
| Glycerin | 5 | Moisturizing agent |
| 1,3-Butanediol | 2 | Moisturizing agent |
| Trehalose | 2 | Moisturizing agent |
| Glycereth-26 | 2 | Moisturizing agent |
| Allantoin | 0.2 | Moisturizing agent |
| Niacinamide | 1 | Lightening agent |
| α-Arbutin | 0.5 | Lightening agent |
| Ascorbyl glucoside | 0.5 | Lightening agent |
| Xanthan gum | 1.5 | Thickening agent |
| Phenoxyethanol | 0.5 | Preservative |
| Isopropyl myristate | 3 | Permeation enhancer |
| Grapefruit essential oil | 0.015 | Essence |
| Water | Balance | |

To a beaker containing an appropriate amount of water, 5% *Radix Astragali* extract, 2% aloe extract, 2% green tea extract, 0.5% sodium hyaluronate, 5% glycerin, 2% 1,3-butanediol, 2% trehalose, 2% glycereth-26, 0.2% allantoin, 1% niacinamide, 0.5% α-arbutin, 0.5% ascorbyl glucoside, 1.5% xanthan gum, 0.5% phenoxyethanol, 3% isopropyl myristate, and 0.015% grapefruit essential oil were added one by one, in which the materials were added with continuous stirring. The materials were stirred evenly, then homogenized for 5 minutes, and filled into a clean container to obtain a skin care product composition.

### Example 2: Preparation of skin care product composition

| Ingredient | Amount (%) | Function |
|---|---|---|
| *Radix Astragali* extract | 12 | / |
| Aloe extract | 2 | / |
| Green tea extract | 2 | / |
| Sodium hyaluronate | 0.5 | Moisturizing agent |
| Glycerin | 10 | Moisturizing agent |
| 1,3-Butanediol | 8 | Moisturizing agent |
| Allantoin | 1 | Moisturizing agent |
| Niacinamide | 1 | Lightening agent |
| α-Arbutin | 0.5 | Lightening agent |
| Ascorbyl glucoside | 0.5 | Lightening agent |
| Xanthan gum | 1 | Thickening agent |
| Phenoxyethanol | 0.5 | Preservative |
| Isopropyl myristate | 5 | Permeation enhancer |
| Grapefruit essential oil | 0.015 | Essence |
| Water | Balance | |

To a beaker containing an appropriate amount of water, 12% *Radix Astragali* extract, 2% aloe extract, 2% green tea extract, 0.5% sodium hyaluronate, 10% glycerin, 8% 1,3-butanediol, 1% allantoin, 1% niacinamide, 0.5% α-arbutin, 0.5% ascorbyl glucoside, 1% xanthan gum, 0.5% phenoxyethanol, 5% isopropyl myristate, and 0.015% grapefruit essential oil were added one by one, in which the materials were added with continuous stirring. The materials were stirred evenly, then homogenized for 5 minutes and filled into a clean container to obtain a skin care product composition.

### Example 3: Preparation of skin care product composition

| Ingredient | Amount (%) | Function |
|---|---|---|
| *Radix Astragali* extract | 4 | / |
| Aloe extract | 1 | / |
| Green tea extract | 1 | / |
| Sodium hyaluronate | 8 | Moisturizing agent |
| Glycerin | 5 | Moisturizing agent |
| 1,3-Butanediol | 5 | Moisturizing agent |
| Allantoin | 0.5 | Moisturizing agent |
| Niacinamide | 5 | Lightening agent |
| α-Arbutin | 2 | Lightening agent |
| Ascorbyl glucoside | 2 | Lightening agent |
| Xanthan gum | 4 | Thickening agent |
| Phenoxyethanol | 2 | Preservative |
| Isopropyl myristate | 7 | Permeation enhancer |
| Grapefruit essential oil | 0.015 | Essence |
| Water | Balance | |

To a beaker containing an appropriate amount of water, 4% *Radix Astragali* extract, 1% aloe extract, 1% green tea extract, 8% sodium hyaluronate, 5% glycerin, 5% 1,3-butanediol, 0.5% allantoin, 5% niacinamide, 2% α-arbutin, 2% ascorbyl glucoside, 4% xanthan gum, 2% phenoxyethanol, 7% isopropyl myristate, and 0.015% grapefruit essential oil were added one by one, in which the materials were added with continuous stirring. The materials were stirred evenly, then homogenized for 5 minutes and filled into a clean container to obtain a skin care product composition.

### Example 4: Preparation of skin care product composition

| Ingredient | Amount (%) | Function |
|---|---|---|
| *Radix Astragali* extract | 10 | / |
| Aloe extract | 2 | / |
| Green tea extract | 2 | / |
| Sodium hyaluronate | 0.5 | Moisturizing agent |
| Glycerin | 5 | Moisturizing agent |
| 1,3-Butanediol | 2 | Moisturizing agent |
| Trehalose | 2 | Moisturizing agent |
| Glycereth-26 | 2 | Moisturizing agent |
| Allantoin | 0.2 | Moisturizing agent |
| Niacinamide | 1 | Lightening agent |
| α-Arbutin | 0.5 | Lightening agent |
| Ascorbyl glucoside | 0.5 | Lightening agent |
| Xanthan gum | 1.5 | Thickening agent |
| Phenoxyethanol | 0.5 | Preservative |
| Isopropyl myristate | 3 | Permeation enhancer |
| Grapefruit essential oil | 0.015 | Essence |
| Water | Balance | |

To a beaker containing an appropriate amount of water, 10% *Radix Astragali* extract, 2% aloe extract, 2% green tea extract, 0.5% sodium hyaluronate, 5% glycerin, 2% 1,3-butanediol, 2% trehalose, 2% glycereth-26, 0.2% allantoin, 1% niacinamide, 0.5% α-arbutin, 0.5% ascorbyl glucoside, 1.5% xanthan gum, 0.5% phenoxyethanol, 3% isopropyl myristate, 0.015% grapefruit essential oil were added one by one, in which the materials were added with continuous stirring. The materials were stirred evenly, then homogenized for 5 minutes and filled into a clean container to obtain a skin care product composition.

### Test example: Evaluation of lightening and moisturizing effects

Volunteers aged between 20 and 40 years old (32 volunteers, including 22 volunteers aged 20 to 30 years old, and 10 volunteers aged 30 to 40 years old) were selected, and the test products were the skin care product compositions of Examples 1, 2, 3, and 4 of the present invention. The test products were applied to the inner side of the left and right arms of the volunteers, left for 20 minutes and then rinsed with clean water. The moisture values of skin after using the test products for 20 minutes, 1 hour, and 2 hours were measured using a skin analyzer (manufacturer: Antsci; model: Skin FX), and the growth values of moisture of skin after using the test products for 20 minutes, 1 hour, and 2 hours were obtained. The test products were applied three times a week. The melanin contents of test areas were measured in the second week, the fourth week, and the eighth week, and the reduction values of melanin content in the second week, the fourth week, and the eighth week were obtained. The growth values of moisture were the means of the growth values of moisture after the products were applied to subjects, and the reduction values of melanin content were the means of the reduction values of melanin content after the products were applied to subjects.

The results of growth values of moisture were shown in the following table:

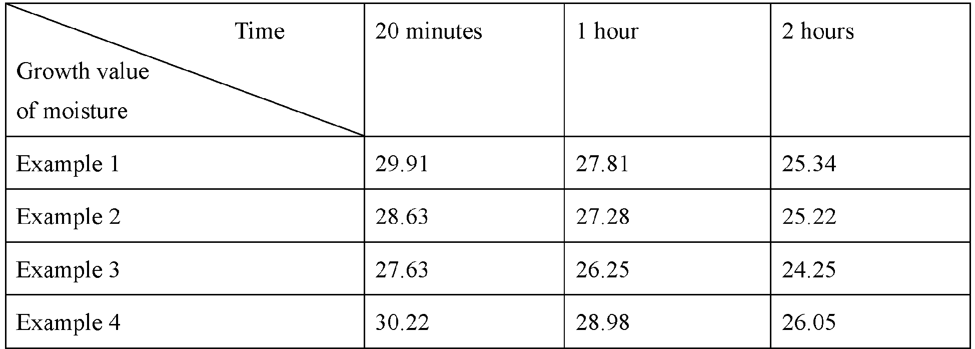

The results of reduction values of melanin content were shown in the following table:

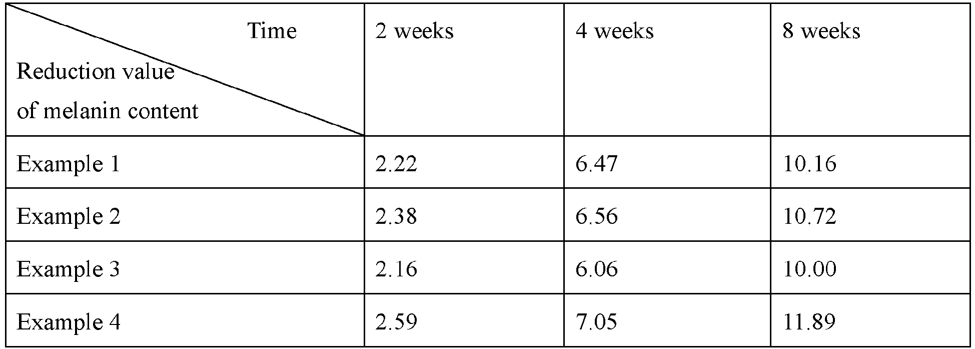

It could be seen from the experimental results that the skin care product compositions of the present invention had excellent lightening and moisturizing effects.

### Comparative Examples 1 to 4

| Ingredient | Amount (%) | | | | |
|---|---|---|---|---|---|
| | Example 4 | Comparative Example 1 | Comparative Example 2 | Comparative Example 3 | Comparative Example 4 |
| *Radix Astragali* extract | 10 | 10 | 10 | 10 | 10 |
| Aloe extract | 2 | 2 | / | / | 2 |
| Green tea extract | 2 | / | 2 | 2 | / |
| *Angelica dahurica* extract | / | 2 | / | / | / |
| *Ligusticum chuanxiong* extract | / | / | 2 | / | / |
| *Ganoderma lucidum* extract | / | / | / | 2 | 2 |
| Sodium hyaluronate | 0.5 | 0.5 | 0.5 | 0.5 | 0.5 |
| Glycerin | 5 | 5 | 5 | 5 | 5 |
| 1,3-Butanediol | 2 | 2 | 2 | 2 | 2 |
| Trehalose | 2 | 2 | 2 | 2 | 2 |
| Glycereth-26 | 2 | 2 | 2 | 2 | 2 |
| Allantoin | 0.2 | 0.2 | 0.2 | 0.2 | 0.2 |
| Niacinamide | 1 | 1 | 1 | 1 | 1 |
| α-Arbutin | 0.5 | 0.5 | 0.5 | 0.5 | 0.5 |
| Ascorbyl glucoside | 0.5 | 0.5 | 0.5 | 0.5 | 0.5 |
| Xanthan gum | 1.5 | 1.5 | 1.5 | 1.5 | 1.5 |
| Phenoxyethanol | 0.5 | 0.5 | 0.5 | 0.5 | 0.5 |
| Isopropyl myristate | 3 | 3 | 3 | 3 | 3 |
| Grapefruit essential oil | 0.015 | 0.015 | 0.015 | 0.015 | 0.015 |
| Water | Balance | Balance | Balance | Balance | Balance |

The ingredients in corresponding amounts were added one by one to a beaker containing an appropriate amount of water, in which the materials were added with continuous stirring. The materials were stirred evenly, then homogenized for 5 minutes, and filled into a clean container to obtain a skin care product composition.

The lightening and moisturizing effects of the skin care product compositions obtained in Comparative Examples 1 to 4 were evaluated (the method was described in detail in the above section of test example).

The results of growth values of moisture were shown in the following table:

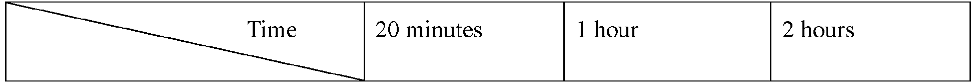

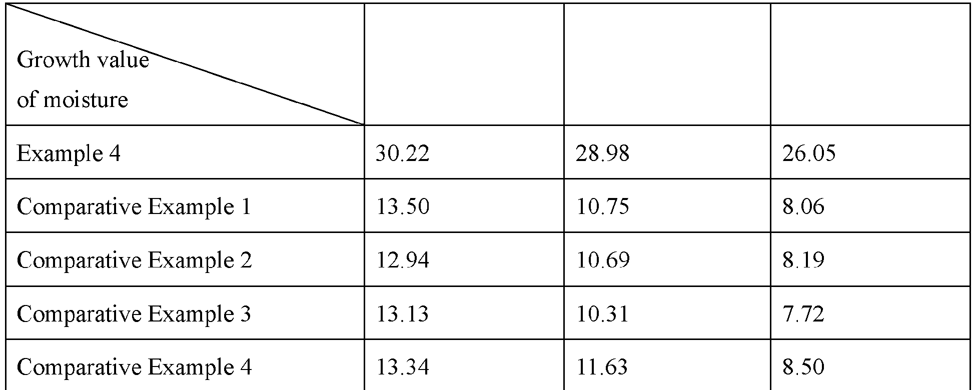

The results of reduction values of melanin content were shown in the following table:

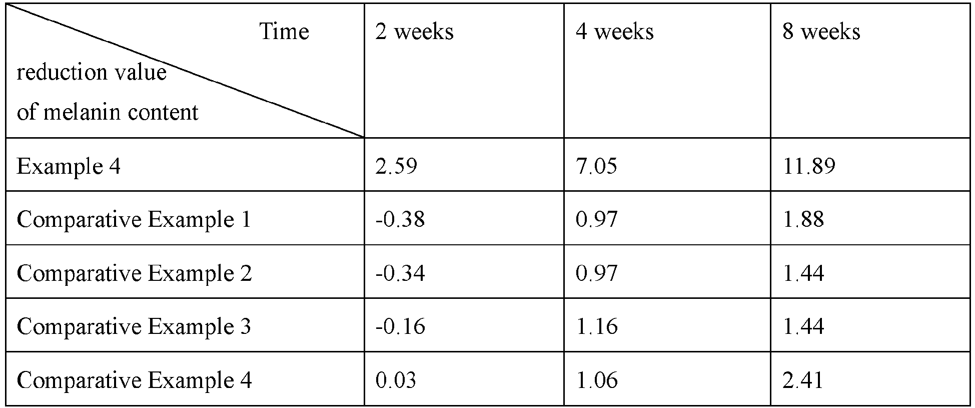

From the above comparison results, it could be seen that in the skin care product compositions of the present invention, only when the three extracts of *Radix Astragali,* aloe and green tea were used in combination according to a certain ratio, significantly better lightening effects could be achieved, and good moisturizing properties could be ensured as well.

### Comparative Examples 5 to 9

| Ingredient | Amount (%) | | | | | |
|---|---|---|---|---|---|---|
| | Example 4 | Comparative Example 5 | Comparative Example 6 | Comparative Example 7 | Comparative Example 8 | Comparative Example 9 |
| *Radix Astragali* extract | 10 | 10 | 10 | 10 | 10 | 10 |
| Aloe extract | 2 | 2 | 2 | 2 | 2 | 2 |
| Green tea extract | 2 | 2 | 2 | 2 | 2 | 2 |
| Sodium hyaluronate | 0.5 | 0.5 | 0.5 | 0.5 | 0.5 | 0.5 |
| Glycerin | 5 | 3 | 0 | 13 | 20 | 1 |
| 1,3-Butanediol | 2 | 2 | 2 | 2 | 2 | 2 |
| Trehalose | 2 | 2 | 2 | 2 | 2 | 2 |
| Glycereth-26 | 2 | 2 | 2 | 2 | 2 | 2 |
| Allantoin | 0.2 | 0.2 | 0.2 | 0.2 | 0.2 | 0.2 |
| Niacinamide | 1 | 1 | 1 | 1 | 1 | 1 |
| a-Arbutin | 0.5 | 0.5 | 0.5 | 0.5 | 0.5 | 0.5 |
| Ascorbyl glucoside | 0.5 | 0.5 | 0.5 | 0.5 | 0.5 | 0.5 |
| Xanthan gum | 1.5 | 1.5 | 1.5 | 1.5 | 1.5 | 1.5 |
| Phenoxyethanol | 0.5 | 0.5 | 0.5 | 0.5 | 0.5 | 0.5 |
| Isopropyl myristate | 3 | 3 | 3 | 3 | 3 | 3 |
| Grapefruit essential oil | 0.015 | 0.015 | 0.015 | 0.015 | 0.015 | 0.015 |
| Water | Balance | Balance | Balance | Balance | Balance | Balance |

The ingredients in corresponding amounts were added one by one to a beaker containing an appropriate amount of water, in which the materials were added with continuous stirring. The materials were stirred evenly, then homogenized for 5 minutes, and filled into a clean container to obtain a skin care product composition.

The lightening and moisturizing effects of the skin care product compositions obtained in Comparative Examples 5 to 9 were evaluated (the method was described in detail in the above section of test example).

The results of growth values of moisture were shown in the following table:

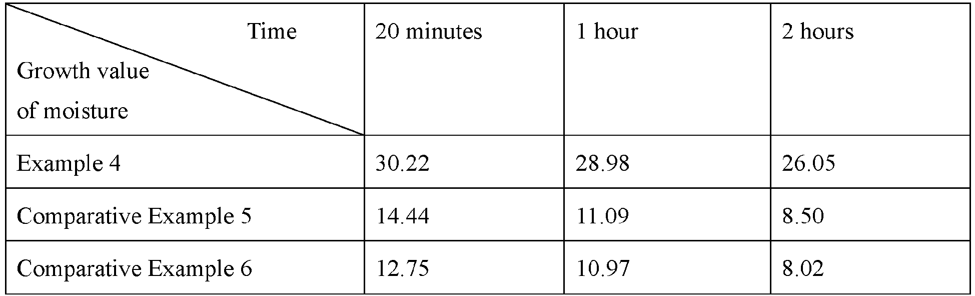

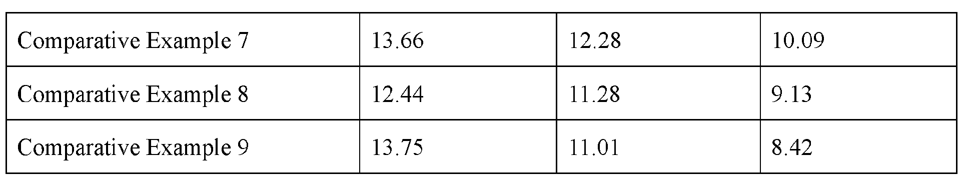

The results of reduction values of melanin content were shown in the following table:

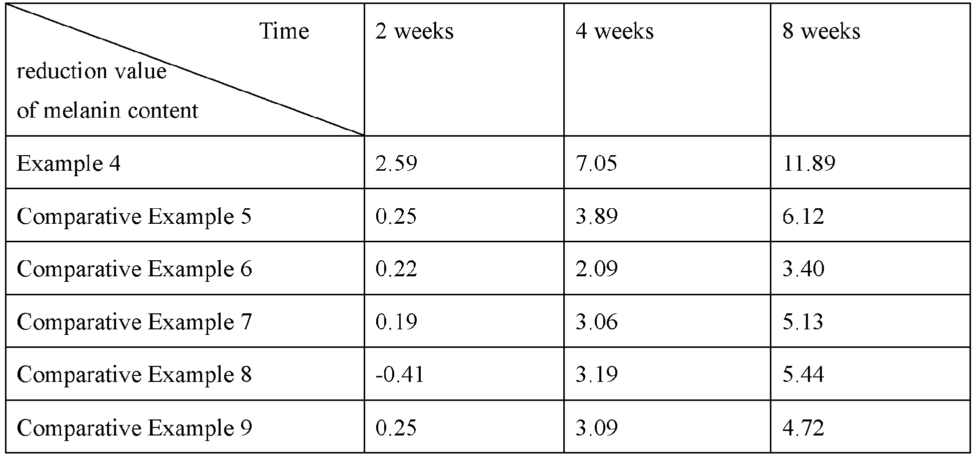

From the above comparison results, it could be seen that in the skin care product compositions of the present invention, when the amount ratio of moisturizing agent was within a specific range, the lightening and moisturizing effects were significantly better.

Finally, it should be noted that for technicians in the technical field to which the present invention belongs, without departing from the concept of the present invention, its architecture can be flexible and changeable, and only simple deduction or replacement should be regarded as falling into the scope of patent protection as determined by the claims of the present invention.

## Claims

1. A skin care product composition, which comprises:
1 to 15 parts by weight of *Radix Astragali* extract;
1 to 5 parts by weight of aloe extract;
1 to 5 parts by weight of green tea extract;
0.1 to 5 parts by weight of thickening agent;
0.1 to 3 parts by weight of preservative.

2. The skin care product composition according to claim 1, which further comprises:
0.1 to 40 parts by weight of moisturizing agent;
0.1 to 30 parts by weight of lightening agent;
0.1 to 10 parts by weight of permeation enhancer.

3. The skin care product composition according to claim 2, which comprises:
4 to 12 parts by weight of *Radix Astragali* extract;
1 to 2 parts by weight of aloe extract;
1 to 2 parts by weight of green tea extract;
0.1 to 5 parts by weight of thickening agent;
0.1 to 3 parts by weight of preservative;
5 to 30 parts by weight of moisturizing agent;
1 to 10 parts by weight of lightening agent;
1 to 7 parts by weight of permeation enhancer.

4. The skin care product composition according to any one of claims 2 to 3, which further comprises:
0.1 to 10 parts by weight (preferably 1 to 7 parts by weight) of emulsifying agent.

5. The skin care product composition according to any one of claims 2 to 4, wherein the moisturizing agent comprises:
0.1 to 30 parts by weight (preferably 5 to 20 parts by weight) of polyol moisturizing agent,
0.1 to 15 parts by weight (preferably 0.1 to 10 parts by weight) of carbohydrate moisturizing agent, and,
0.1 to 30 parts by weight (preferably 0.2 to 2.5 parts by weight) of other moisturizing agent;
preferably, the polyol moisturizing agent is selected from the group consisting of glycerin, 1,3-butanediol, sorbitol, polyethylene glycol, propylene glycol, glycereth-26, and any combination thereof;
more preferably, the polyol moisturizing agent is selected from the group consisting of glycerin, 1,3-butanediol, glycereth-26, and any combination thereof;
most preferably, the polyol moisturizing agent is a composition consisting of 5 to 12 parts by weight of glycerin and 2 to 8 parts by weight of 1,3-butanediol, or a composition consisting of 5 to 12 parts by weight of glycerin, 2 to 8 parts by weight of 1,3-butanediol and 1.5 to 2.5 parts by weight (preferably 2 parts by weight) of glycereth-26;
preferably, the carbohydrate moisturizing agent is selected from the group consisting of sodium hyaluronate, trehalose, tremella polysaccharide, methyl glucose polyether, and any combination thereof;
more preferably, the carbohydrate moisturizing agent is selected from the group consisting of sodium hyaluronate, trehalose, and any combination thereof;
most preferably, the carbohydrate moisturizing agent is a composition consisting of 0.5 to 8 parts by weight of sodium hyaluronate, or a composition consisting of 0.5 to 8 parts by weight of sodium hyaluronate and 1.5 to 2.5 parts by weight (preferably 2 parts by weight) of trehalose;
preferably, the other moisturizing agent is selected from the group consisting of panthenol, ceramide, allantoin, natural plant extract, and any combination thereof;
more preferably, the other moisturizing agent is allantoin;
most preferably, the other moisturizing agent consists of 0.2 to 1 parts by weight of allantoin.

6. The skin care product composition according to claim 5, wherein the moisturizing agent further comprises:
0.1 to 8 parts by weight (preferably 0.1 to 6 parts by weight) of natural moisturizing factor moisturizing agent;
preferably, the natural moisturizing factor moisturizing agent is selected from the group consisting of urea, amino acid, vitamin B6, and any combination thereof.

7. The skin care product composition according to any one of claims 1 to 4, wherein:
the thickening agent is selected from the group consisting of xanthan gum, carbomer 940, polyacrylic acid polymer, and any combination thereof;
preferably, the thickening agent is xanthan gum;
or, the preservative is selected from the group consisting of paraben (e.g., methyl paraben, ethyl paraben, propyl paraben, butyl paraben), phenoxyethanol, benzoic acid/benzyl alcohol and derivatives thereof, and any combination thereof;
preferably, the preservative is phenoxyethanol;
or, the lightening agent is selected from the group consisting of hydroquinone, arbutin, vitamin C and derivatives thereof (e.g., vitamin C, ascorbyl glucoside), niacinamide, natural plant extract, and any combination thereof;
preferably, the lightening agent is selected from the group consisting of arbutin, vitamin C and derivatives thereof (e.g., vitamin C, ascorbyl glucoside), niacinamide, natural plant extract, and any combination thereof;
more preferably, the lightening agent is selected from the group consisting of arbutin, vitamin C and derivatives thereof (e.g., vitamin C, ascorbyl glucoside), niacinamide, and any combination thereof;
or, the permeation enhancer is selected from the group consisting of Azone, isosorbide dimethyl ether, inositol, isopropyl myristate, and any combination thereof;
preferably, the permeation enhancer is isopropyl myristate;
or, the emulsifying agent is selected from the group consisting of glyceryl stearate, methyl glucose sesquistearate, PEG-20 methyl glucose sesquistearate, laureth-7/C₁₃₋₁₄ alkane/polyacrylamide (also known as Emulsifier 305), isopropyl myristate, and any combination thereof;
or, the skin care product composition further comprises an essence;
preferably, the essence is selected from the group consisting of bluebell essence, Miss COCO essence, cherry blossom essence, ebony agarwood essence, jasmine essence, rose essence, and grapefruit essential oil.

8. The skin care product composition according to any one of claims 1 to 7, which consists of:
| Ingredient | Amount (%) |
|---|---|
| *Radix Astragali* extract | 5 |
| aloe extract | 2 |
| green tea extract | 2 |
| sodium hyaluronate | 0.5 |
| glycerin | 5 |
| 1,3-butanediol | 2 |
| trehalose | 2 |
| glycereth-26 | 2 |
| allantoin | 0.2 |
| niacinamide | 1 |
| α-arbutin | 0.5 |
| ascorbyl glucoside | 0.5 |
| xanthan gum | 1.5 |
| phenoxyethanol | 0.5 |
| isopropyl myristate | 3 |
| grapefruit essential oil | 0.015 |
| water | balance |
or,
| Ingredient | Amount (%) |
|---|---|
| *Radix Astragali* extract | 12 |
| aloe extract | 2 |
| green tea extract | 2 |
| sodium hyaluronate | 0.5 |
| glycerin | 10 |
| 1,3-butanediol | 8 |
| allantoin | 1 |
| niacinamide | 1 |
| α-arbutin | 0.5 |
| ascorbyl glucoside | 0.5 |
| xanthan gum | 1 |
| phenoxyethanol | 0.5 |
| isopropyl myristate | 5 |
| grapefruit essential oil | 0.015 |
| water | balance |
or,
| Ingredient | Amount (%) |
|---|---|
| *Radix Astragali* extract | 4 |
| aloe extract | 1 |
| green tea extract | 1 |
| sodium hyaluronate | 8 |
| glycerin | 5 |
| 1,3-butanediol | 5 |
| allantoin | 0.5 |
| niacinamide | 5 |
| α-arbutin | 2 |
| ascorbyl glucoside | 2 |
| xanthan gum | 4 |
| phenoxyethanol | 2 |
| isopropyl myristate | 7 |
| grapefruit essential oil | 0.015 |
| water | balance |
or,
| Ingredient | Amount (%) |
|---|---|
| *Radix Astragali* extract | 10 |
| aloe extract | 2 |
| green tea extract | 2 |
| sodium hyaluronate | 0.5 |
| glycerin | 5 |
| 1,3-butanediol | 2 |
| trehalose | 2 |
| glycereth-26 | 2 |
| allantoin | 0.2 |
| niacinamide | 1 |
| α-arbutin | 0.5 |
| ascorbyl glucoside | 0.5 |
| xanthan gum | 1.5 |
| phenoxyethanol | 0.5 |
| isopropyl myristate | 3 |
| grapefruit essential oil | 0.015 |
| water | balance |

9. A skin care product, which comprises: the skin care product composition according to any one of claims 1 to 8;
preferably, the skin care product is a facial mask.

10. A method for enhancing the effects of lightening and moisturizing the skin, which comprises:
applying the skin care product composition according to any one of claims 1 to 8 or the skin care product according to claim 9 to the skin.
